# EUROPEAN PATENT APPLICATION

(11) **EP 4 368 718 A1**
(43) Date of publication of application: **15.05.2024**
(21) Application number: 22837704.0
(22) Date of filing: 06.07.2022
(51) Int. Cl.: C12N 15/63, C12N 5/079, C12N 5/10

(54) **MICROGLIAL PROGENITOR CELLS, METHOD FOR MANUFACTURING MICROGLIA, AND MANUFACTURED MICROGLIAL PROGENITOR CELLS AND MICROGLIA**

(30) Priority: 06.07.2021 US 202163218502 P
(71) Applicant: Keio University, Tokyo, 108-8345 (JP)
(72) Inventor: SONN, Iki, Tokyo 160-8582 (JP); OKANO, Hideyuki, Tokyo 160-8582 (JP); WATANABE, Hirotaka, Tokyo 160-8582 (JP); MORIMOTO, Satoru, Tokyo 160-8582 (JP)
(74) Representative: Cabinet Becker et Associés
(86) International application number: PCT/JP2022/026843
(87) International publication number: WO 2023/282290

(57) **Abstract**

Provided is a method for producing microglia, including: Step (S1) of inducing differentiation of hemangioblasts to obtain microglial progenitor cells; and Step (S2) of inducing differentiation of the microglial progenitor cells to obtain microglia, in which, in the step of obtaining microglial progenitor cells, expression of PU.1 transcription factor encoded by an exogenous gene is induced, and culture is carried out in the presence of FGF2, SCF, IL-3, IL-6, VEGF, and Wnt inhibitor.

## Description

### [Technical Field]

The present invention relates to microglial progenitor cells, a method for producing microglia, and produced microglial progenitor cells and microglia.

Priority is claimed on United States Patent No. 63/218,502, provisionally filed in the United States July 6, 2021, the content of which is incorporated herein by reference.

### [Background Art]

The brain is composed of nerve cells and glial cells. Microglia are a type of glial cells and account for approximately 5% to 15% of cells in the central nervous system. Microglia are known to function as immunocompetent cells in the central nervous system. In addition to the immune function, microglia also play a variety of physiological functions. In addition, it has become clear that recently identified risk factor genes for neurodegenerative diseases are highly expressed in microglia. For the above reasons, microglia are becoming increasingly important as a research subject.

It is difficult to obtain a sufficient amount of microglia from living bodies as experimental materials. A method for artificially producing microglia in vitro can be thought as an alternative. Microglia are derived from mesoderm, and pluripotent stem cells differentiate into microglia through hemangioblasts and microglial progenitor cells.

For example, Non-Patent Document 1 discloses that human-induced pluripotent stem cells (hiPSCs) are cultured with a plurality of cytokines over a long period of time, and target cells are separated and collected through cell sorting or the like to produce human microglia-like cells.

### [Citation List]

### [Non-Patent Document]

[Non-Patent Document 1]
Abud E. M., et al., iPSC-Derived Human Microglia-like Cells to Study Neurological Diseases, Neuron 2017; 94:278-765, 293.e9

### [Summary of Invention]

### [Technical Problem]

However, the method described in Non-Patent Document 1 and similar methods have room for improvement from the viewpoints of efficiency (qualitatively, quantitatively, and periodically) and costs.

Therefore, an object of the present invention is to provide a highly efficient and inexpensive method for producing microglia, a method for producing microglial progenitor cells for use in the production method, and microglia that enable subculture and function as microglia.

### [Solution to Problem]

The present invention has the following aspects.
[1] A method for producing microglia, including: a step of inducing differentiation of hemangioblasts to obtain microglial progenitor cells; and a step of inducing differentiation of the microglial progenitor cells to obtain microglia, in which, in the step of obtaining microglial progenitor cells, expression of PU.1 transcription factor encoded by an exogenous gene is induced, and culture is carried out in the presence of FGF2, SCF, IL-3, IL-6, VEGF, and Wnt inhibitor.
[2] The method for producing microglia according to [1], in which transcription of the exogenous gene under control of an inducible promoter is induced to induce the expression.
[3] The method for producing microglia according to [1] or [2], in which, in the step of obtaining microglia, the microglial progenitor cells are cultured in a medium containing IL-34, TGFβ1, M-CSF, CD200, and CX3CL1.
[4] The method for producing microglia according to any one of [1] to [3], in which the hemangioblasts are cells induced to differentiate from pluripotent stem cells.
[5] The method for producing microglia according to [4], in which the pluripotent stem cells are of human origin.
[6] The method for producing microglia according to any one of [1] to [5], in which the hemangioblasts are produced by a step of inducing differentiation of pluripotent stem cells to obtain hemangioblasts, and the step of obtaining hemangioblasts includes: a step (1) of culturing pluripotent stem cells in a medium containing BMP4 and CHIR99021; a step (2) of culturing the cells after the step (1) in a medium containing BMP4, VEGF, and FGF2; and a step (3) of culturing the cells after the step (2) in a medium containing VEGF and FGF2.
[7] The method for producing microglia according to any one of [1] to [6], in which the induction of the expression of the PU.1 transcription factor in the step of obtaining microglial progenitor cells is performed by transiently inducing expression of the PU.1 transcription factor.
[8] Microglial progenitor cells to be subjected to differentiation induction to produce microglia exhibiting characteristics of intracerebral microglia, in which the microglial progenitor cells are produced by inducing expression of PU.1 transcription factor encoded by an exogenous gene in the hemangioblasts and carrying out culture in the presence of FGF2, SCF, IL-3, IL-6, VEGF, and Wnt inhibitor.
[9] Microglia which are produced by inducing differentiation of the microglial progenitor cells according to [8].

### [Advantageous Effects of Invention]

By the production method of the present invention, it is possible to produce microglia-like cells having properties closer to intracerebral microglia with high efficiency, at low cost, and in a short period of time.

### [Brief Description of Drawings]

FIG. 1 is a flowchart showing a method for producing microglia according to the present embodiment.
FIG. 2 is a flowchart showing a method for producing hemangioblasts from pluripotent stem cells.
FIG. 3 is a micrograph showing microglial progenitor cells.
FIG. 4 is a micrograph showing microglia according to the present embodiment.
FIG. 5 shows parts of three types of plasmids which are gene constructs used in the present embodiment.
FIG. 6 shows graphs evaluating expression levels of microglia-specific markers.
FIG. 7 shows a graph evaluating a comprehensive transcription profile through principal component analysis (PCA).
FIG. 8 shows graphs showing the influence of LPS stimulation on expression levels of inflammatory cytokines.

### [Description of Embodiments]

### [Notation of gene name and protein name]

In the present specification, human genes and human proteins will be denoted by uppercase letters. In addition, mouse genes will be denoted by an uppercase letter for the first letter and lowercase letters for the rest. In addition, mouse proteins are denoted by uppercase letters. However, in some cases, human genes, mouse genes, and genes of other species may be expressed without strictly distinguishing them. In addition, human proteins, mouse proteins, and proteins of other species may be expressed without strictly distinguishing them.

### [Method for producing microglia]

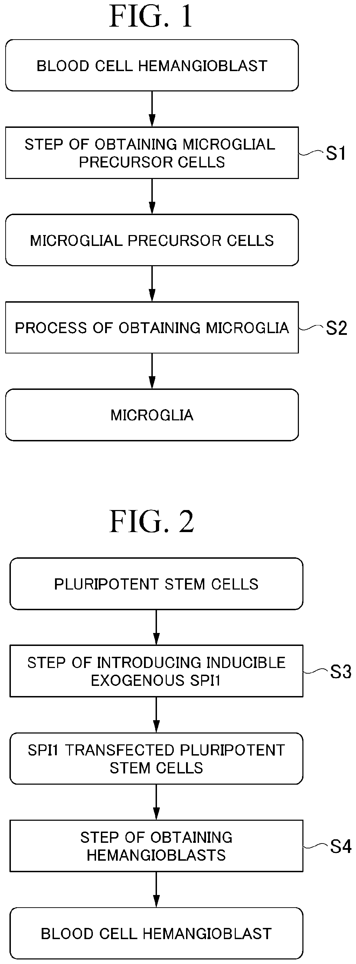

FIG. 1 is a flowchart showing a method for producing microglia according to one embodiment.

In one embodiment, the present invention provides a method for producing microglia, including: Step S1 of inducing differentiation of hemangioblasts to obtain microglial progenitor cells; and Step S2 of inducing differentiation of the microglial progenitor cells to obtain microglia, in which, in the step of obtaining microglial progenitor cells, expression of PU.1 transcription factor encoded by an exogenous gene is induced.

As will be described in examples, the present inventors have revealed that microglia (artificial microglia-like cells) can be produced with high efficiency, at low cost, and in a short period of time by obtaining microglial progenitor cells and inducing differentiation of the microglial progenitor cells through the method for producing microglia of the present embodiment.

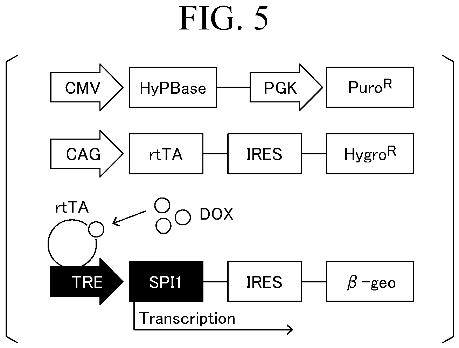

Hemangioblasts used as starting materials in the present embodiment can be produced through, for example, a method for producing hemangioblasts to be described below. Modules (for example, plasmids containing HyPBase, rtTA, and SP11 shown in FIG. 5) necessary for inducible expression of exogenous PU.1 transcription factor have been preliminarily genetically introduced into the hemangioblasts used in the present embodiment.

Exogenous genes refer to genes which are not endogenous genes but are additionally provided to cells through gene introduction. A base sequence of a protein-encoding region may be the same as that of a corresponding endogenous gene, or may have mutations such as amino acid substitutions, insertions, and deletions. In addition, exogenous genes may be derived from heterologous organisms.

A non-coding region of an exogenous gene may be linked to 5'UTR (untranslated region) 3'UTR or 5'UTR and 3'UTR required for artificial induction of expression.

The induction of expression of an exogenous gene refers to artificial induction of expression of an exogenous gene. Specifically, by turning on an inducible promoter of an exogenous gene (in the present embodiment, the SP11 gene encoding the PU.1 transcription factor) that is under control of the inducible promoter, the amount of exogenous gene transcribed is increased and expression of gene products is induced.

In Step S1 of obtaining microglial progenitor cells, expression of PU.1 transcription factor may be induced by inducing transcription of the exogenous SPI1 gene under control of the inducible promoter. Accordingly, microglial progenitor cells that are more suitable for producing microglia are easily produced.

As inducible promoters, drug-inducible promoters can be used. As drug-inducible promoters, promoters whose expression induction is turned on and off by addition of doxycycline can be used. In addition, temperature-sensitive inducible promoters can be used.

In the present embodiment, as inducible promoters, promoters which have a TRE sequence and whose expression induction is turned on by addition of doxycycline can be used.

By artificially inducing expression of an exogenous gene, the expression level of the exogenous gene at a transcription level increases more than twice as much as when the expression is not induced. The degree of increase may be 5 times or more, 10 times or more, 25 times or more, 50 times or more, 100 times or more, or 200 times or more. The upper limit of the degree of increase is not particularly limited but may be 1,000 times or less.

The expression level at the transcription level can be evaluated through, for example, quantitative reverse transcription PCR (qRT-PCR) using one obtained by reverse-transcribing mRNA prepared from target cells as a template.

Step S1 of obtaining microglial progenitor cells includes Step S1a of inducing expression of PU.1 transcription factor in the presence of FGF2, SCF, IL-3, IL-6, VEGF, and IWR-le. By inducing differentiation from microglial progenitor cells obtained through Step S1a into microglia, it is possible to appropriately induce differentiation from hemangioblasts into microglial progenitor cells and to produce microglial progenitor cells suitable for producing microglia.

Step S1 of obtaining microglial progenitor cells preferably includes Step S1b of subjecting the cells after Step S1a of inducing expression of PU.1 transcription factor in the presence of FGF2, SCF, IL-3, IL-6, VEGF, and IWR-le to induction of expression of PU.1 transcription factor in the presence of FGF2, SCF, IL-3, and IL-6.

As a medium in Step S1 of obtaining microglial progenitor cells, it is possible to use StemPro^{™}-34 SFM (1×) (Gibco^{™}, 1063011) supplemented with GlutaMax (Gibco^{™}, 1×), holo-transferrin (Naacalai Tesque, 20 µg/mL), L-ascorbic acid (Sigma-Aldrich Co. LLC., 500 µM), monothioglycerol (Sigma-Aldrich Co. LLC., 450 µM), and penicillin/streptomycin and passed through a 0.45 um filter (hereinafter referred to as "hiHPC diff. medium").

The concentration of FGF2 may be in a range of 5 ng/mL plus or minus 30% or plus or minus 15%.

The concentration of SCF may be in a range of 50 ng/mL plus or minus 30% or plus or minus 15%.

The concentration of IL-3 may be in a range of 30 ng/mL plus or minus 30% or plus or minus 15%.

The concentration of IL-6 may be in a range of 10 ng/mL plus or minus 30% or plus or minus 15%.

The concentration of VEGF may be in a range of 10 ng/mL plus or minus 30% or plus or minus 15%.

The concentration of IWR-le may be in a range of 5 µM plus or minus 30% or plus or minus 15%.

The concentration of doxycycline may be in a range of 1 µg/mL plus or minus 30% or plus or minus 15%.

Step S2 of inducing differentiation of microglial progenitor cells to obtain microglia preferably includes Step S2a of culturing the microglial progenitor cells obtained in Step S1 of obtaining microglial progenitor cells in a medium containing IL-34, TGFβ1, M-CSF, CD200, and CX3CL1. Accordingly, microglia (microglia-like cells) having properties similar to intracerebral microglia are likely to be efficiently produced from microglial progenitor cells.

As a medium in Step S2 of obtaining microglia, it is possible to use DMEM/F12 (1:1, Gibco^{™}) supplemented with GlutaMax (Gibco^{™}, 1×), NEAA (Thermo Fisher Scientific Inc., 1×), B27 (Thermo Fisher Scientific Inc., 1×), N2 (Thermo Fisher Scientific Inc., 0.5×), ITS-G (Thermo Fisher Scientific Inc., 2%), insulin (Sigma-Aldrich Co. LLC., 5 µg/mL), monothioglycerol (Sigma-Aldrich Co. LLC., 200 µM), and penicillin/streptomycin and passed through a 0.45 um filter. DMEM can also be replaced with the same amount of IMDM (Gibco^{™}) (hereinafter referred to as "hiMGL diff. medium").

The concentration of IL-34 may be in a range of 100 ng/mL plus or minus 30% or plus or minus 15%.

The concentration of TGFβ1 may be in a range of 50 ng/mL plus or minus 30% or plus or minus 15%.

The concentration of M-CSF may be in a range of 25 ng/mL plus or minus 30% or plus or minus 15%.

The concentration of CD200 may be in a range of 100 ng/mL plus or minus 30% or plus or minus 15%.

The concentration of CX3CL1 may be in a range of 100 ng/mL plus or minus 30% or plus or minus 15%.

### [Method for producing hemangioblasts]

FIG. 2 is a flowchart showing a method for producing hemangioblasts from pluripotent stem cells.

Hemangioblasts used as starting materials in the present embodiment include Step S3 of introducing exogenous gene SPI1 under control of an inducible promoter into pluripotent stem cells and S4 of inducing differentiation of the pluripotent stem cells into which SP11 is introduced to obtain hemangioblasts.

The SPI1 gene is a gene encoding PU.1 transcription factor.

The above-described pluripotent stem cells may be, for example, ES cells or induced pluripotent stem cells (iPSCs). In addition, the above-described pluripotent stem cells may be human-derived cells or may be cells derived from non-human animals such as mice, rats, pigs, goats, sheep, and monkeys.

If the pluripotent stem cells are human-derived cells, microglia-like cells suitable as experimental materials in human microglia research can be prepared with high efficiency, at low cost, and in a short period of time.

In addition, the above-described pluripotent stem cells may be induced pluripotent stem cells derived from a healthy subject or may be induced pluripotent stem cells derived from a patient with a neurological disease. In a case where microglia are produced from induced pluripotent stem cells derived from a patient with a neurological disease, the obtained microglia can be used as a neurological disease model. Such microglia are useful for elucidating mechanisms of neurological diseases.

In Step S3 of introducing exogenous gene SPI1 into pluripotent stem cells, a module (for example, a gene construct to be described below) necessary for inducible expression of exogenous PU.1 transcription factor is genetically introduced (transfected) into pluripotent stem cells.

Gene introduction is performed, for example, using Genejuice (Sigma-Aldrich Co. LLC., 70967) as a transfection reagent according to the instruction manual.

Pluripotent stem cells may be cultured and maintained feeder-free.

Step S4 of obtaining hemangioblasts preferably includes Step S4a of culturing pluripotent stem cells in a medium containing BMP4 and CHIR99021, Step S4b of culturing the cells after Step S4a in a medium containing BMP4, VEGF, and FGF2, and Step S4c of culturing the cells after Step S4b in a medium containing VEGF and FGF2.

Accordingly, hemangioblasts suitable for starting materials of the present embodiment can be produced.

hiHPC diff. medium can be used as a medium in Step S4 of obtaining hemangioblasts.

The concentration of BMP4 may be in a range of 20 ng/mL plus or minus 30% or plus or minus 15%.

The concentration of CHIR99021 may be in a range of 2 µM plus or minus 30% or plus or minus 15%.

The concentration of VEGF may be in a range of 50 ng/mL plus or minus 30% or plus or minus 15%.

The concentration of FGF2 may be in a range of 20 ng/mL plus or minus 30% or plus or minus 15%.

The concentration of VEGF in Step S4c may be in a range of 15 ng/mL plus or minus 30% or plus or minus 15%.

The concentration of FGF2 in Step S4c may be in a range of 5 ng/mL plus or minus 30% or plus or minus 15%.

### [Microglial progenitor cells and microglia]

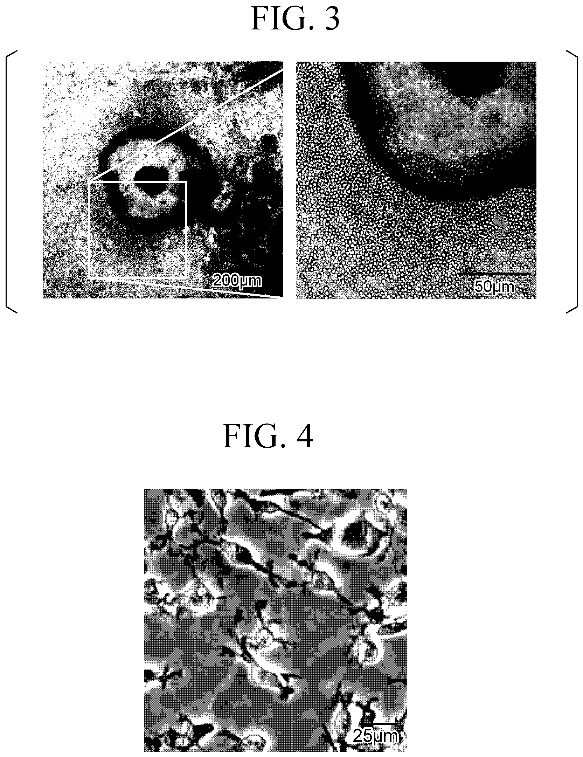

FIG. 3 is a micrograph showing microglial progenitor cells.

The large mass in the center is a hemangioblast, and the many small granular cells around it are microglial progenitor cells.

FIG. 4 is a micrograph showing microglia according to one embodiment.

In one embodiment, the present invention provides microglial progenitor cells which are produced by inducing expression of PU.1 transcription factor encoded by an exogenous gene in hemangioblasts and are to be subjected to differentiation induction to produce microglia exhibiting characteristics of intracerebral microglia.

In addition, in one embodiment, the present invention provides microglia which are produced by inducing differentiation of the microglial progenitor cells according to one embodiment described above and exhibit characteristics of intracerebral microglia.

In the related art, it is not known that it is possible to produce microglia-like cells more efficiently by inducing differentiation of hemangioblasts into microglial progenitor cells while inducing expression of PU.1 transcription factor during production of the microglia-like cells.

On the other hand, microglial progenitor cells can be produced through the above-described production method and microglia can be produced through the above-described method in which the microglial progenitor cells are used as starting materials to more efficiently produce microglia-like cells.

The microglial progenitor cells of the present embodiment are floating cells, double positive for CD11b/CD45, and positive for CD235a.

The microglia of the present embodiment are adherent cells, positive for 1BA1 and CX3CR, and positive for TMEM119 (or P2RY12). In microglia, the expression levels of DAP12 and TREM2 are high.

The microglial progenitor cells and microglia of the present embodiment are induced to differentiate from pluripotent stem cells into which inducible exogenous SPI1 is introduced. For this reason, by evaluating the presence or absence of this inducible exogenous SPI1 as genetic information, it is possible to confirm whether those are derived from hemangioblasts obtained through Step S3 of introducing exogenous gene SP11 and Step S4 of obtaining hemangioblasts shown in FIG. 2.

The above-described evaluation can be performed, for example, through PCR using a forward primer complementary to the exogenous DNA fragment and a reverse primer complementary to the SPI1 coding sequence using the total DNA of a target cell as a template. In addition, as primers, a forward primer complementary to the SPI1 coding sequence and a reverse primer complementary to the exogenous DNA fragment can also be used.

The microglial progenitor cells and microglia of the present embodiment may be housed in a container at a cell density of 1 × 10⁵ cells/mL or more, for example, 1 × 10⁴ cells/mL or more or 1 × 10⁶ cells/mL or more.

The microglial progenitor cells and microglia of the present embodiment are in a frozen state, and may be housed in a cryotube or the like that is normally used for cryopreservation of cells. In this case, a commercially available cryopreservation liquid can be used. Examples of serum-free cryopreservation liquids include CELLBANKER (registered trademark) 2 (Nippon Zenyaku Kogyo Co., Ltd.) or Banbanker (registered trademark) (Lymphotec Inc.).

### [Gene construct]

FIG. 5 shows parts of three types of plasmids which are gene constructs used in the present embodiment. FIG. 5 shows, in order from the top, a part containing a hyperactive piggyBac transposase (HyPBase) coding region and a puromycin resistance gene, a part containing a reverse tetracycline transactivator (rtTA) coding region and a hygromycin resistance gene, and a part containing a lacZ-neomycin fusion gene (β-geo) and an exogenous PU.1 coding region under control of an inducible promoter.

The gene construct shown at the top is used in combination for the purpose of improving gene introduction efficiency, and is not essential.

The gene construct shown in the center contains a gene encoding rtTA, a factor that binds to a TRE sequence and promotes transcription of downstream genes in the presence of doxycycline. This gene construct is necessary in a case of inducing expression of PU. 1 transcription factor with a doxycycline-inducible promoter.

The gene construct is unnecessary in a case where inducible promoter other than the doxycycline-inducible promoter is used.

These gene constructs can be used in the above-described method for producing microglia or the above-described method for producing microglial progenitor cells.

In the gene constructs used in the present embodiment, the SP11 gene is linked downstream of a promoter which has a TRE sequence and whose expression induction is turned on by addition of doxycycline.

In a case where doxycycline is added to a culture medium, it is taken into cells and binds to rtTA. Doxycycline-bound rtTA binds to the TRE sequence and promotes transcription of SPI1.

An IRES sequence contributing to efficient translation of transcripts and a β-geo sequence functioning as a selection marker are linked downstream of SP11.

### [Examples]

Examples will be shown below to describe the present invention in more detail, but the present invention is not limited to the following examples.

### [Experimental Example 1]

### (Establishment of SPI1 -introduced human pluripotent stem cell line)

A human-induced pluripotent stem cell (hiPSC) line in which exogenous SPI1 was introduced under control of an inducible promoter was established.

### <<Maintenance and culture of human-induced pluripotent stem cells>>

hiPSC line 201B7 (Cell 2007; 131:861-872), WD39 (Mol. Brain 2012; 5: 35), and RPC802 (ReproCell Inc., cat# RCRP002N) were cultured feeder-free. These iPSCs were maintained in a 12-well plate (Corning Inc.) coated with iMatrix-511. StemFit/AK02N (Ajinomoto) supplemented with penicillin and streptomycin was used as a medium. A humidified incubator (37°C, 5% CO₂) was used for culture. iPSCs were subcultured weekly, and the medium was replaced every 2 days.

Other methods followed the protocol published by CiRA (https://www.cira.kyoto-u.ac.jp/j/research/img/protocol/).

### «Establishment of human induced pluripotent stem cell line into which exogenous SPI1 is introduced»

Genejuice (Sigma-Aldrich Co. LLC., 70967) was used as a transfection reagent. 9 µL of Genejuice was mixed with 200 µL of Opti-MEM medium (Gibco^{™}, 31985070), and the mixture was allowed to stand at room temperature for 5 minutes.

Thereafter, the plasmids including the expression modules shown in FIG. 5 (pCMV-HyPBase_PGK-Puro (0.4 µg), pG-PB-CAG-rtTA3G-IH (0.4 µg), and PB-tet-PHS-SPI1 (0.8 µg)) were added thereto, and the mixture was left to stand at room temperature for another 5 minutes.

The human pluripotent stem cells cultured feeder-free were treated with Y-27632 (Wako, 253-00511 (10 µM/AK02N)) for 1 hour.

Thereafter, the pluripotent stem cells were detached from the surface of the culture container using TrypLE Select (Life Technogogies, A12859-01, 0.5×, incubated at 37°C for 10 minutes). After centrifugation at 1,000 rpm for 5 minutes, the collected cells were stained with trypan blue, and the number of live cells was counted.

After centrifugation, the supernatant was removed, the above-described Genejuice-DNA mixture was added, and the mixture was allowed to stand at room temperature for 10 minutes.

Thereafter, the cells were evenly seeded in 6 wells of a 12-well plate. 0.8 mL of AK02N supplemented with penicillin/streptomycin, Y-27632 (10 µm), and iMatrix0511 was added to the plate. On the next day, the medium was replaced and Y-27632 was removed from the culture liquid. Hygromaycin (20 µg/mL) was added to the replaced medium to select human iPSCs.

After colonies grew larger, puromycin (2 µg/mL) was added to the medium. The final concentrations of hygromycin and puromycin were 200 µg/mL and 10 µg/mL, respectively.

The colonies were mechanically isolated using a P10 tip under a microscope. The isolated colonies were individually seeded in a 24-well plate with a medium supplemented with Y-27632 and iMatrix-511.

Several clones were selected from each of the cell lines RPC802, 201B7, and WD39 by selection with puromycin and hygromycin. These clones were confirmed to be human induced pluripotent stem cell lines into which exogenous SPI1 was introduced.

Human microglia were induced to differentiate from the human induced pluripotent stem cell lines established using the above-described three types of cell lines.

A technique obtained by modifying a technique disclosed in well-known literature was used as a basic hiMGL production technique (hereinafter referred to as "CK protocol"). The contents of the well-known literature, Dev Biol 2015; 407:1-11 and Stem Cell Reports 2019; 13:515-529, are incorporated herein.

In contrast to the CK protocol, a hiMGL production method further involving inducible expression of exogenous SPI1 heredity is hereinafter referred to as "PU protocol."

The production of microglia from iPSCs can be broadly divided into two steps.

The first step corresponds to day 0 to day 18 from the start of differentiation induction. In the first step, differentiation is induced from human pluripotent stem cells to hemangioblasts and from the hemangioblasts to microglial progenitor cells.

The second step corresponds to day 19 or beyond. In the second stroke, differentiation is induced from the microglial progenitor cells to microglia.

### [Experimental Example 2]

### (Induction of differentiation from human pluripotent stem cells to hemangioblasts)

Differentiation was induced from the established human induced pluripotent stem cell lines to hemangioblasts.

### <<Induction of differentiation from hiPSCs to hemangioblasts (days 0 to 6)»

Days 0 to 4 correspond to the period of induction of differentiation from hiPSCs to mesodermal cells.

Days 4 to 6 correspond to the period of induction of differentiation from the mesodermal cells to hemangioblasts.

Differentiation induction was started when hiPSC colonies reached an appropriate size (usually around 5 days after subculture of hiPSCs).

On day 0, hiPSCs on a 6-well plate into which exogenous SPI1 was introduced were washed once with PBS. Thereafter, 2 mL of hiHPC diff. medium supplemented with BMP4 (PeproTech, 20 ng/mL) and CRIR9901 (Focus Biomolecules, 2 µM) was added to each well. The cells were allowed to stand in a hypoxic incubator (5% O₂, 5% CO₂, 37°C) until day 6. The cells subcultured while maintaining the colony size smaller than normal.

On day 2, the entire amount of medium was replaced with 2 mL of a fresh medium supplemented with BMP4 (20 ng/mL), VEGF (Thermo Fisher Scientific Inc., 50 ng/mL), and FGF2 (PeproTech, 20 ng/mL).

On day 4, the entire amount of medium was replaced with 2 mL of a fresh medium supplemented with VEGF (15 ng/mL) and FGF2 (5 ng/mL).

On day 6, the cells were collected into a 15-mL tube and centrifuged at 300 g at room temperature for 5 minutes. Thereafter, half of the medium was removed from the supernatant, and approximately 1 mL of the remaining medium containing the collected cells was returned to the wells. hiHPC diff. medium supplemented with 1 mL of VEGF (30 ng/mL), FGF2 (10 ng/mL), SCF (PeproTech, 100 ng/mL), IL-3 (PeproTech, 60 ng/mL), IL-6 (PeproTech, 20 ng/mL), and Wnt Inhibitor (IWR-1e, Thermo Fisher Scientific Inc., 5 µM) was added to each well.

After day 6, the cells were allowed to stand in a normoxic incubator (20% O₂, 5% CO₂, 37°C).

When the morphology of the cells on day 6 was observed under a microscope, the outer edges of the hiPSC cell aggregations became unclear, and the presence of small cells dispersed around the hiPCS cell aggregations was observed. The above-described cell aggregations whose outer edges became unclear are hemangioblasts.

In the PU protocol, doxycycline (1 µg/mL) was added to the culture liquid from day 6 to day 18 from the start of culture.

By adding doxycycline, the expression level of the SP11 gene evaluated through qRT-PCR increased by at least about 50 times compared to a case without doxycycline.

In a case of performing a similar evaluation using a primer set that selectively amplifies endogenous SP11 gene, the expression level of the SP11 gene was significantly low regardless of the presence or absence of doxycycline (approximately 1-2 times).

### [Experimental Example 3]

### (Induction of differentiation from hemangioblasts to microglial progenitor cells)

### <<Induction of differentiation from hemangioblasts to microglial progenitor cells (on days 7 to 18)»

Days 7 to 14 correspond to the period of induction of differentiation from the hemangioblasts to primary hematopoietic stem cells.

Days 14 to 18 correspond to the period of induction of differentiation from primary hematopoietic stem cells to microglial progenitor cells.

On day 9, the medium was replenished in the same way as on day 6.

On day 12, the entire amount of medium containing floating cells was collected into a 15-mL tube and centrifuged at 300 g at room temperature for 5 minutes. Thereafter, half of the medium was carefully removed from the supernatant, and approximately 1 mL of the collected cell-containing medium was returned to the wells. 2 mL of hiHPC diff. medium supplemented with FGF2 (10 ng/mL), SCF (100 ng/mL), IL-3 (60 ng/mL), and IL-6 (20 ng/mL) was added to each well.

On day 14, the medium was replenished in the same way as on day 12.

On day 18, the cells were collected in a falcon tube and centrifuged at 300 × g for 5 minutes. Thereafter, the supernatant was carefully and completely removed.

When the morphology of the cells on days 15 to 18 was observed under a microscope, the presence of a large amount of small granular (bubble-like) cells was confirmed. These granular cells were considered to be microglial progenitor cells induced to differentiate from hemangioblasts.

By inducing differentiation from hemangioblasts to microglial progenitor cells in a state where expression of the exogenous SP11 gene was induced in the presence of an appropriate factor, a larger amount (at least twofold) of floating microglial precursor cells can be obtained compared to comparable preparations prepared according to the CK protocol.

The cells on days 4, 8, 12, and 16 from the start of differentiation induction were evaluated for the presence or absence of CD34, CD43, CD11b, and CD45 markers. The evaluation results are shown in Table 1. As controls, evaluation results of cultured cells without addition of doxycycline and evaluation results of cultured cells without addition of doxycycline and Wnt Inhibitor (IWR-1e) are shown.

Regarding the CD34/CD43 markers, no significant differences were observed in contrast to the control cells, but regarding the CD11b/CD45 markers, significant differences were observed in contrast to the control cells.

**[Table 1]**

| | CD34⁺/CD43⁺ (%) | CD11b⁺/CD45⁺ (%) |
|---|---|---|
| D4 | 85.82 | 3.86 |
| D8 | 86.76 | 4.0 |
| D12 | 95.86 | 18.73 |
| D16 | 99.27 | 91.265 |
| No Dox | 93.02 | 60.060 |
| No Dox/o IRW-1e | 83.77 | 27.610 |

| | | |
|---|---|---|
| *: The numerical values are average values where n is 3 or more. | | |

When CD235a and KDR markers were similarly evaluated, significant differences were observed in contrast to the control cells.

### [Experimental Example 4]

### (Induction of differentiation to human microglia derived from human-induced pluripotent stem cells)

Human microglia were induced to differentiate from microglial progenitor cells.

### <<Induction of differentiation from microglial progenitor cells to microglia (from day 19)»

Day 19 or beyond corresponds to a maturation period of microglia.

On day 18, the cells were collected into a 15-mL tube and centrifuged at 300 g at room temperature for 5 minutes. Thereafter, the supernatant was removed, and the collected cells (microglial progenitor cells) were evenly seeded in a plurality of wells through the method described below.

The microglial progenitor cells were seeded in a 12-well plate at 40,000 to 50,000 cells in 0.8 mL of hiMGL diff. medium. The medium used was hiMGL diff. medium supplemented with three kinds of cytokine cocktails (IL-34 100 ng/mL; TCFβ1 50 ng/mL; and M-CSF 25 ng/mL (all are PeproTech)). The plate used was one coated with Poly-D-Lysine (PDL, R&D Systems) at least 1 hour before seeding and washed three times with PBS.

Under the above-described conditions, microglial progenitor cells were induced to differentiate into microglia from day 19.

After day 19, half of the medium was replaced every 3 days. After this, the cultured hiMGLs for 1 week can be used for subsequent analysis.

The medium used after day 19 was one supplemented with five kinds of cytokine cocktails (IL-34 100 ng/mL; TGFβ1 50 ng/mL; and M-CSF 25 ng/mL, CD200 100 ng/mL; and CX3CL1 100 ng/mL (all are PeproTech)).

### «Morphological observation of microglia»

When the morphology of the cells on day 25 was confirmed under a microscope, a morphology characteristic of intracerebral microglia could be confirmed. When the culture was continued further (on day 39), further cell branching was recognized as shown in FIG. 4.

Hereinafter, microglia-like cells obtained through the above-described method will be referred to as human-induced microglia-like cells (hiMGLs).

### <<Confirmation of marker expression of microglia>>

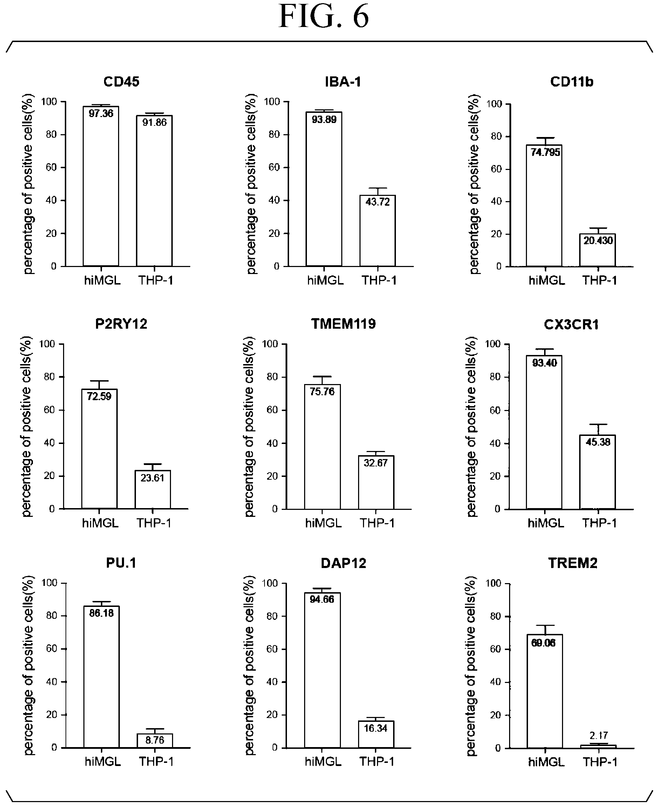

Marker expression of the obtained microglia (microglia-like cells) was confirmed. THP-1, a human monocyte cell line, was used for comparison. Marker expression of CD45, IBA1, CD11b, P2RY12, TMEM119, CR3CR1, PU.1, DAP12, and TREM2 was analyzed through flow cytometry. The results are shown in Fig. 6. CD45 is a hematopoietic stem cell marker, IBA1, PU.1, and CX3CR1 are myeloid lineage markers, and TMEM119 and P2RY12 are microglia-specific markers.

The CD45 positive cell rate was at the same level between hiMGL and THP-1. On the other hand, the positive cell rates for IBA1, CD11b, P2RY12, TMEM119, CR3CR1, PU.1, DAP12, and TREM2 were significantly higher in hiMGL than in THP-1.

In addition, cells were immunostained for microglia-specific markers 1BA1, TMEM119, CX3CR1, P2RY12, DAP12, and TREM2. Immunostaining was also performed in combination with PU.1 and Hoechst staining, and a merged image was created.

Most hiMGLs expressed these microglia-specific markers, and the presence of PU.1 was confirmed in the nucleus.

In addition, Western blotting confirmed that the protein amounts of DAP10 and DAP12 were significantly increased in microglia obtained through induction of expression of PU.1.

The above-described results mean that microglia-like cells expressing microglia-specific markers could be produced in a highly pure state and in a short period of time using the PU protocol.

### <<Comprehensive analysis of expression patterns of microglia>>

The effect of induction of SPI1 gene expression on transcriptional patterns of produced microglia was analyzed through comprehensive transcriptional analysis. Through this analysis, it is possible to evaluate to what extent the produced microglia resemble intracerebral microglia.

Poly(A)+ RNA was prepared from target cells and converted into cDNA library fragments (average length: 350 bp). Adapters were linked to both ends of the cross section for base sequence determination. A KAPA mRNA capture Kit (KK8440, Kapa Biosystems), a KAPA RNA HyperPrep Kit (KK8542, Kapa Biosystems), a KAPA Pure Beads (KK8443, Kapa Biosystems), and a SeqCap Adapter Kit A (Roche) were used according to the attached instruction manual.

The cDNA library was quantified using a KAPA Library Quantification Kit (KK4828, Kapa Biosystems). Illumina HiSqX was used for sequence determination.

The quality of mRNA-seq was confirmed, and those with low reliability were excluded from data to be analyzed using Trim Galore! (ver. 0.4.0).

The data were mapped to Homo sapiens (Hg19) genome using Hisat2 (ver. 2.2.2.2.0). The output summary data was processed using DESeq2 (3.3.0) to exclude ones that were not mentioned in any of the materials. Finally, normalization was performed using a variance-stabilizing transformation (VST).

Regarding Monocytes, AH1 iMGL, adult microglia, and fetus microglia in mRNA-seq analysis, deposited data from previous studies were also used (GSE89189 in GEO&DDBJ).

This analysis data was deposited in the Gene Expression Omnibus of NCB1 under accession no. GSE178284.

Expression profiles of the cells produced using the CK protocol and PU protocol on day 25 after induction of differentiation were obtained through the above-described method and compared with expression profiles of the deposited monocytes, adult primary human microglia, fetus primary human microglia, and other hiPSC-derived microglia-like cells.

The obtained data were analyzed through principal component analysis (PCA) and hierarchical cluster analysis as a comprehensive transcription profile evaluation.

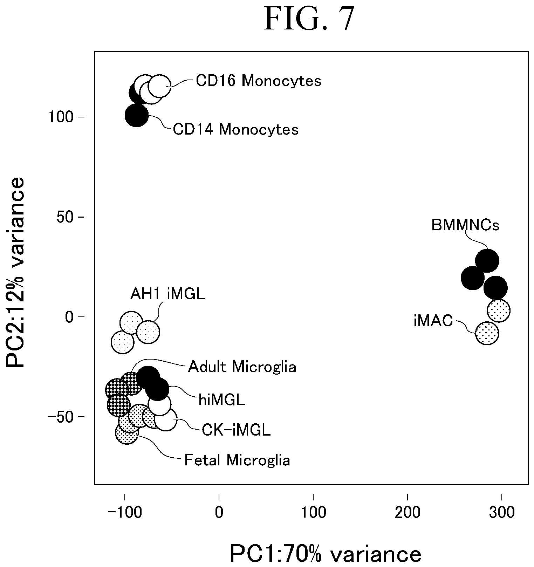

FIG. 7 shows a part of the above-described analysis and shows analysis results of PCA. These analysis results showed that the microglia (microglia-like cells) produced using the PU protocol have an expression pattern significantly similar to intracerebral microglia.

### «Evaluation of physiological function of microglia»

As an evaluation of physiological functions of the microglia produced using the PU protocol, phagocytic ability, inflammatory cytokine secretion ability, and inflammasome generation ability were evaluated.

Latex beads and fibrillar Aβ-42 were used for evaluating phagocytic ability.

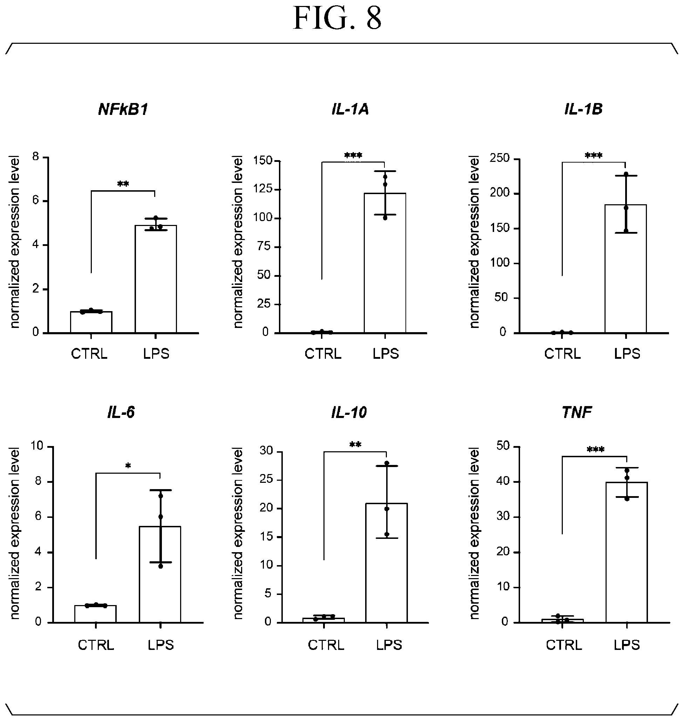

The expression levels of NKkB1, IL-1A, IL-1B, IL-6, IL-10, and TNF as inflammatory cytokines were evaluated based on the presence or absence of stimulation with lipopolysaccharides (LPS) (FIG. 8).

Like intracerebral microglia, the microglia produced using the PU protocol were shown to have phagocytic ability, inflammatory cytokine secretion ability, and inflammasome generation ability.

### «Co-culture with mouse neuron primary cells (in vitro analysis)»

The microglia produced using the PU protocol were co-cultured with mouse neuron primary cells (derived from the hippocampus), and change in microglia morphology, increase in cytoplasmic area of the microglia, and expression of microglia-specific markers were evaluated.

The microglia produced using the PU protocol changed from an amoeboid shape to a branched shape when co-cultured with mouse neuron primary cells. In addition, the cytoplasmic area of the microglia was significantly increased. Furthermore, expression of IBA1, a microglia-specific marker, was also recognized.

In addition, it was confirmed that co-culture with hiMGLs promoted neuron maturation and spine formation in mouse neuron primary cells.

From the above, it is thought that the hiMGLs produced using the PU protocol can interact with mouse neuron primary cells and can appropriately reproduce intracerebral microglia with respect to specific functions. The hiMGLs produced using the PU protocol are thought to be useful experimental materials for studying functions of microglia.

### «Co-culture with mouse neuron primary cells (in vivo analysis)»

Intracerebral microglia of a living mouse were replaced with microglia derived from hiPSCs produced using the PU protocol through nasal transplantation, and colonization of the microglia and change in cell morphology were observed.

PLX5622 was administered to the mouse (8 weeks old after birth, wild type C57BL/6J mouse) for 1 week starting 8 days before transplantation. Thereafter, normal diet was administered for 24 hours.

Thereafter, 2.5 µL of hyaluronidase (manufacturer, catalog number) was injected into left and right nostrils of the mouse at a 5-min interval.

1 hour later, 1×10⁶ hiMGLs were injected through the left nostril of the mouse, and 3 minutes later, 1 × 10⁶ hiMGLs were injected through the right nostril.

10 minutes later, 1 × 10⁶ hiMGLs were injected through the left nostril of the mouse, and 3 minutes later, 1 × 10⁶ hiMGLs were injected through the right nostril. 24 hours later, hiMGLs were repeatedly injected through the nostrils.

Thereafter, cytokine hCSF1 (250 ng/mL) and hTGF-β1 (100 ng/mL) were injected through the nostrils of the mouse every 12 hours.

14 days after the start of transplantation, brain tissue of the mouse was excised, and the prepared slice sample was subjected to immunohistological staining.

In a negative control specimen derived from a mouse without transplantation, no expression of a human microglia marker IBA1 and a human cytoplasmic marker STEM121 was observed.

On the other hand, in the specimen derived from the mouse with transplantation, the presence of cells expressing IBA1 and STEM121 was confirmed in the olfactory bulb, cortex, hippocampus, striatum, and cerebellum.

It was confirmed that these cells expressing IBA1 and STEM121 had a branched shape.

From the above, it is thought that, in the in vivo experiment using the living mouse, the hiMGLs produced using the PU protocol can interact with mouse neuron primary cells and can appropriately reproduce intracerebral microglia with respect to specific functions. The hiMGLs produced using the PU protocol are thought to be useful experimental materials for studying functions of microglia.

### [Industrial Applicability]

It is possible to produce microglia-like cells having properties closer to intracerebral microglia with high efficiency, at low cost, and in a short period of time.

## Claims

1. A method for producing microglia, comprising:
a step of inducing differentiation of hemangioblasts to obtain microglial progenitor cells; and
a step of inducing differentiation of the microglial progenitor cells to obtain microglia,
wherein, in the step of obtaining microglial progenitor cells, expression of PU.1 transcription factor encoded by an exogenous gene is induced, and culture is carried out in the presence of FGF2, SCF, IL-3, IL-6, VEGF, and Wnt inhibitor.

2. The method for producing microglia according to claim 1,
wherein transcription of the exogenous gene under control of an inducible promoter is induced to induce the expression.

3. The method for producing microglia according to claim 1,
wherein, in the step of obtaining microglia, the microglial progenitor cells are cultured in a medium containing IL-34, TGFβ1, M-CSF, CD200, and CX3CL1.

4. The method for producing microglia according to claim 1,
wherein the hemangioblasts are cells induced to differentiate from pluripotent stem cells.

5. The method for producing microglia according to claim 4,
wherein the pluripotent stem cells are of human origin.

6. The method for producing microglia according to any one of claims 1 to 5,
wherein the hemangioblasts are produced by a step of inducing differentiation of pluripotent stem cells to obtain hemangioblasts, and
wherein the step of obtaining hemangioblasts includes:
a step (1) of culturing pluripotent stem cells in a medium containing BMP4 and CHIR99021:
a step (2) of culturing the cells after the step (1) in a medium containing BMP4, VEGF, and FGF2; and
a step (3) of culturing the cells after the step (2) in a medium containing VEGF and FGF2.

7. The method for producing microglia according to claim 1,
wherein the induction of the expression of the PU.1 transcription factor in the step of obtaining microglial progenitor cells is performed by transiently inducing expression of the PU.1 transcription factor.

8. Microglial progenitor cells to be subjected to differentiation induction to produce microglia exhibiting characteristics of intracerebral microglia,
wherein, the microglial progenitor cells are produced by inducing expression of PU.1 transcription factor encoded by an exogenous gene in the hemangioblasts and carrying out culture in the presence of FGF2, SCF, IL-3, IL-6, VEGF, and Wnt inhibitor.

9. Microglia which are produced by inducing differentiation of the microglial progenitor cells according to claim 8 and exhibit characteristics of intracerebral microglia.
